# EUROPEAN PATENT SPECIFICATION

(11) **EP 3 067 093 B1**
(45) Date of publication and mention of the grant of the patent: **07.08.2019**
(21) Application number: 15382111.1
(22) Date of filing: 12.03.2015
(51) Int. Cl.: A61P 19/08, A61K 31/192, A61K 38/16, A61K 31/4245, A61K 31/433, A61K 31/4468, A61K 31/46

(54) **USE OF AN INHIBITOR OF ADRENOMEDULLIN FOR THE MANUFACTURE OF A DRUG USEFUL IN THE PREVENTION AND TREATMENT OF DISEASES THAT REDUCE BONE DENSITY**
VERWENDUNG EINES INHIBITORS VON ADRENOMEDULLIN ZUR HERSTELLUNG EINES ARZNEIMITTELS ZUR PRÄVENTION UND BEHANDLUNG VON KNOCHENDICHTEVERRINGERNDEN KRANKHEITEN
UTILISATION D'UN INHIBITEUR D'ADRÉNOMÉDULLINE POUR LA FABRICATION D'UN MÉDICAMENT UTILE DANS LA PRÉVENTION ET LE TRAITEMENT DE MALADIES QUI RÉDUISENT LA DENSITÉ OSSEUSE

(43) Date of publication of application: 14.09.2016
(73) Proprietor: Fundación Rioja Salud, 26006 Logroño (La Rioja) (ES); Universidad De Zaragoza, 50009 Zaragoza (ES); Centro de Investigación Biomédica en Red, 28029 Madrid (ES)
(72) Inventor: Martínez Ramírez, Alfredo, 26006 Logroño ( La Rioja) (ES); Martínez Herrero, Sonia, 26006 Logroño ( La Rioja) (ES); Larráyoz Roldán, Ignacio, 26006 Logroño ( La Rioja) (ES); Ochoa Callejero, Laura, 26006 Logroño ( La Rioja) (ES); Fernández Ledesma, Luis José, 50009 Zaragoza (ES); Ochoa Garrido, Ignacio, 28029 Madrid (ES); Sanmartín, Josune García, 26006 Logroño (La Rioja) (ES)
(74) Representative: Carvajal y Urquijo, Isabel

(56) References cited:
- WO-A1-00/58348
- WO-A1-97/07214
- WO-A2-99/16406
- WO-A2-2005/026741
- BENJAMIN UZAN ET AL: "Adrenomedullin is anti-apoptotic in osteoblasts through CGRP1 receptors and MEK-ERK pathway", JOURNAL OF CELLULAR PHYSIOLOGY, vol. 215, no. 1, 1 April 2008 (2008-04-01) , pages 122-128, XP055193101, ISSN: 0021-9541, DOI: 10.1002/jcp.21294
- J. CORNISH ET AL: "Adrenomedullin-a regulator of bone formation", REGULATORY PEPTIDES, vol. 112, no. 1-3, 1 April 2003 (2003-04-01), pages 79-86, XP055192819, ISSN: 0167-0115, DOI: 10.1016/S0167-0115(03)00025-9

### SEQUENCE LISTING

<110> FUNDACION RIOJA SALUD
<120> USE OF AN INHIBITOR OF ADRENOMEDULLIN FOR THE MANUFACTURE OF A DRUG USEFUL IN THE PREVENTION AND TREATMENT OF DISEASES THAT REDUCE BONE DENSITY
<160> 3
<170> BiSSAP 1.2
<210> 1
   <211> 31
   <212> PRT
   <213> Artificial Sequence
<220>
   <223> Adrenomedullin fragment AM 22-52
<220>
   <221> MOD_RES
   <222> 31
   <223> AMIDATION
<400> 1
   Thr Val Gln Lys Leu Ala His Gln Ile Tyr Gln Phe Thr Asp Lys Asp
   1               5                   10                  15
   Lys Asp Asn Val Ala Pro Arg Ser Lys Ile Ser Pro Gln Gly Tyr
               20                  25                  30
<210> 2
   <211> 21
   SEQUENCE LISTING
<110> FUNDACION RIOJA SALUD
<120> USE OF AN INHIBITOR OF ADRENOMEDULLIN FOR THE MANUFACTURE OF A DRUG USEFUL
   IN THE PREVENTION AND TREATMENT OF DISEASES THAT REDUCE BONE DENSITY
<160> 3
<170> BiSSAP 1.2
<210> 1
   <211> 31
   <212> PRT
   <213> Artificial Sequence
<220>
   <223> Adrenomedullin fragment AM 22-52
<220>
   <221> MOD_RES
   <222> 31
   <223> AMIDATION
<400> 1
   Thr Val Gln Lys Leu Ala His Gln Ile Tyr Gln Phe Thr Asp Lys Asp
   1               5                   10                  15
   Lys Asp Asn Val Ala Pro Arg Ser Lys Ile Ser Pro Gln Gly Tyr
               20                  25                  30
<210> 2
   <211> 21
   <212> RNA
   <213> Artificial Sequence
<220>
   <221> source
   <222> 1..21
   <223> /mol_type="unassigned RNA" /note="Adrenomedullin target sequence 1" /organism="Artificial Sequence"
<400> 2
   ggcacaccag atctaccagt t 21
<210> 3
   <211> 21
   <212> RNA
   <213> Artificial Sequence
<220>
   <221> source
   <222> 1..21
   <223> /mol_type="unassigned RNA" /note="Adrenomedullin target sequence 2" /organism="Artificial Sequence"
<400> 3
   ggactctggt gtcttctaag c 21

## Description

### Field of the invention

The present invention is related to the pharmaceutical industry in general and in particular to inhibitors of adrenomedullin or inhibitors of adrenomedullin receptors for the manufacture of a drug useful in the prevention and treatment of diseases that reduce bone density in a human or animal.

### Background of the invention

Osteoporosis is a serious problem in the elderly population and is characterized by a decrease in bone mineral density (a T scale of mineral density lower than 2.5) and deterioration of bone micro architecture, which can lead to an increased risk of fractures. Osteoporosis is more common among women, especially after menopause. Estrogen deficiency increases the bone resorption process, causing the balance between bone deposition by osteoblasts and resorption by osteoclasts inclined to net bone loss. Many studies have shown that hormone replacement therapy can be used to prevent and/or treat osteoporosis. However, long-term estrogen supplementation has severe risks including endometrial cancer, breast cancer or ovarian cancer, as well as cardiovascular risks, so this treatment is no longer recommended.

It has been suggested that estrogens may inhibit osteoclast activity via the PTH and calcitonin levels, so that these hormones could be used to maintain bone balance. On the other hand, estrogens inhibit bone resorption by inhibiting the secretion of important cytokines such as interleukins IL-1 and IL-6. Furthermore, both estrogens and IL-1 and IL-6 regulate the production of prostaglandins through activation of COX-2, which in turn modulates bone resorption. Currently, the treatment of osteoporosis is advancing from conventional monotherapies towards new regimes of combined treatments. However, patients may benefit from the emergence of new preventive and/or therapeutic drugs that act through different routes, not yet explored.

AM is a 52 amino acid peptide originally identified in an extract from a human tumor, but it has subsequently been observed that it is produced in many parts of the body and has numerous functions. AM is also capable of regulating the secretion of other hormones, such as insulin. The AM receptor consists of a seven transmembrane domain protein called CLR and another single domain protein called RAMP2 or RAMP3. By using antibodies against AM it has been shown that this peptide is expressed in embryonic primordia of the wings of chicken embryos and in chondrocytes and osteoblasts of the growth plate of bones, suggesting a possible role in bone development. AM binds osteoblasts and induces the growth of osteoblasts and chondrocytes *in vitro.* At the same time, AM reduces the death of serum deprived osteoblasts due to apoptosis. These cellular actions result in a net increase in bone mass in *ex vivo* experiments, but *in vivo* experiments on the action of AM in bone growth have never been carried out.

To conduct such studies it is necessary to have a knockout (KO) mouse model where the AM gene or its receptor has been removed. Several attempts to accomplish this strategy have shown that deletion of this gene from the beginning of development leads to embryonic death due to severe vascular deficiencies. To solve this problem, a conditional knockout model has been developed using the Cre/loxP technology, which allows deleting the gene from a particular cell type, such as neurons or endothelial cells. This technology also allows the construction of an inducible KO where gene deletion occurs by injecting a certain substance into the animal.

### Description of the invention

The present invention provides the use of an inhibitor of adrenomedullin or an inhibitor of adrenomedullin receptors for the manufacture of a drug useful in the prevention and treatment of diseases that reduce bone density in a human or animal, hereinafter, use of the invention.

The use of the invention is also defined as an inhibitor of adrenomedullin or an inhibitor of adrenomedullin receptors for use thereof in the prevention and treatment of diseases that reduce bone density in a human or animal, hereinafter, use of the invention.

The use of the invention is also defined as a method for the prevention and treatment of diseases that reduce bone density in a human or animal, which comprises administering an inhibitor of adrenomedullin or an inhibitor of adrenomedullin receptors in a human or animal.

Inhibitors of the physiological activity of AM, such as monoclonal antibodies, have been described in the state of the art (Martinez A. et al. (1996) Regulation of insulin secretion and blood glucose metabolism by adrenomedullin. Endocrinology 137: 2626-2632). Monoclonal antibodies that block the activity of AM are used. There are also polyclonal antibodies to both the peptide and its receptors, which also act blocking peptide-receptor interaction.

Another way of blocking the activity of AM is the use of peptide fragment AM22-52 (SEQ ID NO: 1) (Ishikawa T. et al. (2003) Adrenomedullin antagonist suppresses in vivo growth of human pancreatic cancer cells in SCID mice by suppressing angiogenesis. Oncogene 22: 1238-1242) which acts as an inhibitor since it binds the receptor but does not trigger the intracellular signaling cascade. The endogenous synthesis of this peptide fragment can also de induced by genetic engineering, by injecting a plasmid that synthesizes said fragment and thus inhibits the action of AM. Molecules that silence the expression of both AM and its receptors can also be used, for example using small RNAs. The inhibition of the expression of the adrenomedullin receptor by interference RNA has been described (Ramachandran V. et al. (2007) Adrenomedullin is expressed in pancreatic cancer and stimulates cell proliferation and invasion in an autocrine manner via the adrenomedullin receptor, ADMR. Cancer Res 67: 2666-2675).

Small size molecules that act as AM inhibitors have been described in document WO2005/026741. One of them, the inhibitor molecule of formula 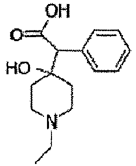 has been used in the examples of the present invention.

In the examples and figures we have referred to this same molecule as molecule 16311.

Furthermore, it can be inferred that a vaccine against AM or its fragments would also be effective in reducing the activity of AM.

The present invention demonstrates that inhibitors of adrenomedullin are useful for the manufacture of a drug useful in the prevention and treatment of diseases that reduce bone density in a human or animal.

Therefore, the inhibitors described in the state of the art are useful for the manufacture of a drug useful in the prevention and treatment of diseases that reduce bone density in a human or animal.

Consequently, another embodiment is the use of the invention, wherein said inhibitor is selected from the group consisting of the fragment of adrenomedullin AM₂₂₋₅₂ identified by the sequence SEQ ID NO: 1 (not part of the claimed invention) and the compounds of formula:

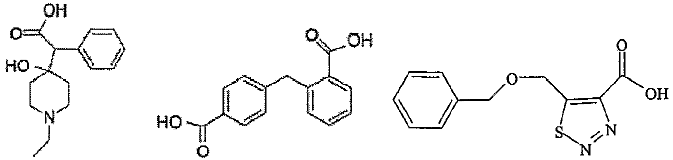

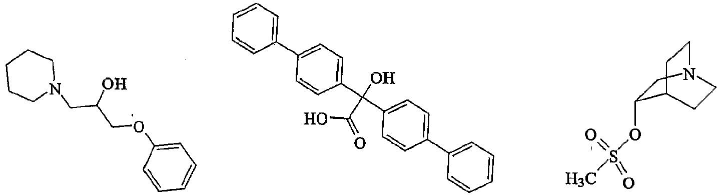

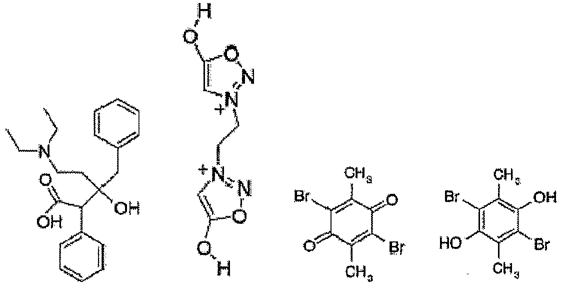

This drug can be used for any other disease where bone density is drastically reduced, such as for example osteomalacia, prolonged immobility, rheumatoid arthritis, chronic kidney disease, hyperparathyroidism, Cushing's disease, gastric bypass, cystic fibrosis, eating disorders, etc.

Therefore, another embodiment is the use of the invention, wherein said disease is selected from the group consisting of osteoporosis, osteomalacia, rheumatoid arthritis, chronic kidney disease, hyperparathyroidism, Cushing's disease, cystic fibrosis, eating disorders, gastric bypass and prolonged immobility.

Another embodiment is the use of the invention wherein said nucleotide is a molecule of interference RNA, which comprises a sequence complementary to SEQ ID NO: 2 or SEQ ID NO: 3.

Another embodiment is the use of the invention, wherein said inhibitor is used in combination with drugs selected from the group consisting of drugs that decrease bone mass or density, drugs that hormonally block prostate or breast cancer, drugs for treating seizures or epilepsy, heparin and glucocorticoids.

The drug can be used in humans and in farm or domestic animals.

Therefore, another embodiment is the use of the invention, wherein said animal is a farm animal or domestic animal.

An inducible knockout (KO) for AM in adult mice has been created in the present invention. These mice live and are an ideal model to test the influence of AM in bone metabolism and osteoporosis. The KO mice have more bone density than wild-type (WT) mice, indicating that, contrary to what was happening in *in vitro* and *ex vivo* models, AM enhances osteoporosis. It has been found that this activity is developed through an indirect mechanism involving inhibition of insulin secretion, hyperglycemia and secretion of ghrelin, which in turn modulates bone metabolism. In order to test the hypothesis that the reduction in the activity of AM can prevent osteoporosis, ovariectomized female mice were treated with a small molecule capable of inhibiting the activity of AM, showing that inhibition of this activity prevents bone loss associated with the lack of estrogen.

The drug can also be used in situations where bone loss depends on the decrease in gravity such as, for example, in space travel.

Therefore, another embodiment is the use of the invention, wherein the reduction of bone mass in a human is due to a state of weightlessness.

Another embodiment is the use of the invention, wherein the weightlessness is due to space travel.

### Free text of sequence listing

The free text that appears in the sequence listing is provided below.
SEQ ID NO: 1. Fragment AM22-52 of adrenomedullin
   31. Amidation (residue 31 is amidated at the C-terminal end).
SEQ ID NO: 2. Adrenomedullin target sequence 1
SEQ ID NO: 3. Adrenomedullin target sequence 2

### Brief description of the drawings

Figure 1. Verification of the deletion of the AM gene after receiving doxycycline. Colon and brain DNA was extracted from various animals with wild (WT) and knockout (KO) genotype. The complete gene is distinguished by a PCR band of about 2500 bp, whereas the deleted gene generates a band of about 600 bp. The positive (C+) and negative (C-) controls are on the right of the image. The molecular weight ladders were loaded on the left.
Figure 2. Evolution of weight in wild type (WT) and AM-deficient (KO) male mice. Doxycycline was applied at weeks 11 to 13. The asterisks indicate statistically significant changes with respect to WT mice. **: p<0.01; ***: p<0.001.
Figure 3. Evolution of weight in particular individuals. In addition to showing a clear difference in weight between WT and KO animals, it is shown how KO animals have a very heterogeneous pattern of increase and decrease in weight throughout their life. These changes are due to the transient accumulation of fluid in their tissues (anasarca).
Figure 4. Femur bone density in wild type (WT) male and in animals lacking the AM gene (KO). The difference is statistically significant (*: p<0.05). The unit represented on the y axis is the Hounsfield unit.
Figure 5. Glucose tolerance curves in wild type (WT) and AM-deficient (KO) male. The KO animals show a clear hyperglycemia. The asterisks indicate statistically significant changes with respect to WT mice. *: p<0.05; ***: p<0.001.
Figure 6. Quantification of ghrelin in the blood of wild type (WT) and AM-deficient (KO) mice. The latter have higher values than control mice. The difference is statistically significant (*: p<0.05).

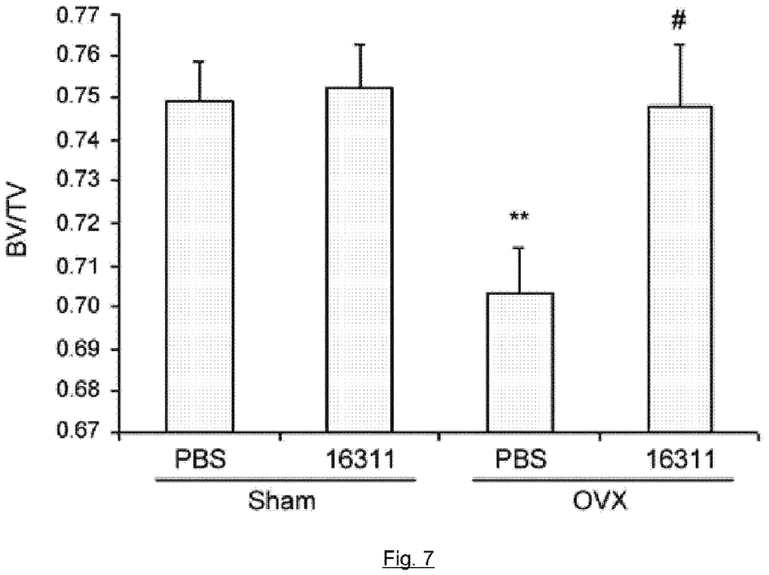

Figure 7. Measurement of femur bone volume in ovariectomized female mice (OVX) or subjected to a Sham operation. Groups 2 and 4 received the inhibitor (molecule 16311). The ovariectomized females (group 3) have lower bone volume (**: p<0.01) than Sham controls (group 1) due to osteoporosis caused by lack of estrogen. Females of group 4 (ovariectomized but treated with 16311) maintained their bone mass without differences with the control group (group 1) and had significantly (#: p<0.05) more bone than those of group 3, indicating that the molecule 16311 prevents osteoporotic effects of lack of estrogens. The data represent the ratio of the volume occupied by the bone matrix to the total volume of bone (BV/TV).

### Modes of preferred embodiment

### Example 1. Creation of a viable inducible KO model.

Mice where the AM gene (*adm*) was surrounded by *loxP* sequences were crossbred with transgenic animals expressing Cre under the control of a tetracycline responsive promoter (tetO, line number 6234, The Jackson Laboratory, Bar Arbor, ME) and with a mutant expressing an optimized version of the reverse transactivator controlled by tetracycline (rtTA-M₂, line number 6965, The Jackson Laboratory). All lines belong to the genetic pool C57BL/6. The tri-transgenic animals are viable. The following genotypes were selected for the experiments: normal controls (homozygous for the *adm* wild allele, and heterozygous for tetO and rtTA-M₂) and KO animals (homozygous for the *adm* "floxed" allele, and heterozygous for tetO and rtTA-M₂). Induction of deletion occurred when animals were exposed to a dose of 2mg/ml of doxycycline (Sigma) in the drinking water for 15 days. All work involving animals was performed in accordance with European (86/609/CEE) and Spanish (RD53/2013) legislation and approved by the Authority Responsible for Animal Welfare of the Biomedical Research Centre of La Rioja, OEBA-CIBIR.

To verify deletion of the gene, DNA was extracted from different organs and subjected to PCR with primers located outside *loxP* sequences; a band of about 2000 bp in the case of the intact gene and of about 600 bp after deletion were expected.

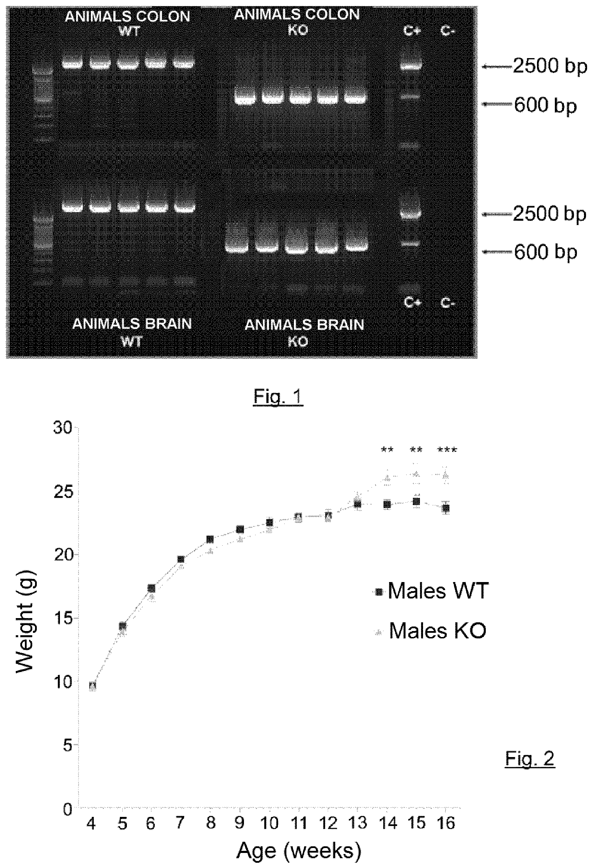

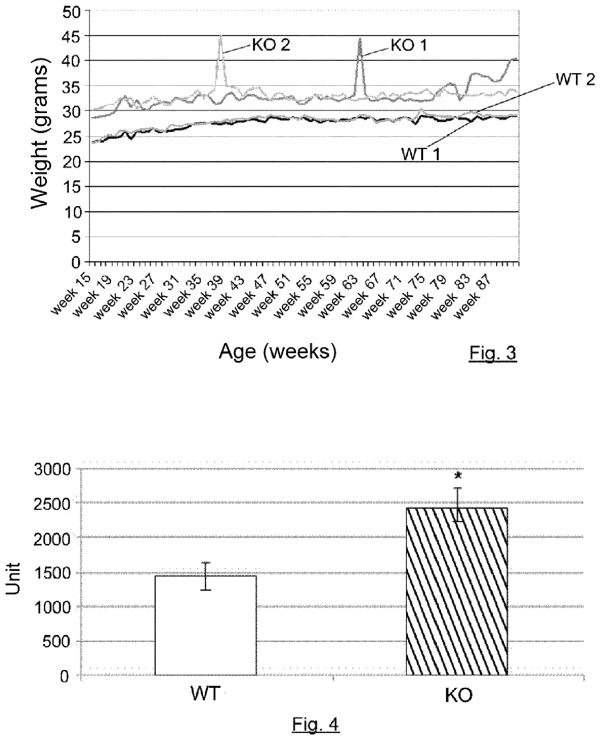

The generated mice survive without problems to deletion induction, having KO mice of more than 100 weeks of age. It has been found by PCR that induced KO mice lose the *adm* gene throughout their organism (Fig. 1). Following the weight of the animals, there is an apparent weight gain in the KO mice when compared with WT siblings (wild type), from the treatment with doxycycline, especially among males (Fig. 2). By studying in detail the individual graphs, it is noted that some KO mice gain a lot of weight in short periods of time, which they then lose as easily; this phenomenon can be seen several times throughout their life (Fig. 3). A more detailed study of these animals leads us to conclude that this increase in weight is due to two reasons: on one hand, to a generalized subcutaneous edema, also called anasarca, which explains the rapid weight changes in both directions. This observation is consistent with that described in embryos where the AM gene was removed, producing fetal hydrops. However, death does not occur in animals of this example, but this phenomenon is regulated spontaneously. On the other hand, by using microCT it has been found that bone density is higher in the KO animals than in WT animals, especially in males (Fig. 4). This leads us to conclude that the absence of AM leads to increased skeletal ossification.

### Example 2. Physiological explanation for the relationship between AM and osteoporosis.

In previous experiments performed *in vitro* or *ex vivo* it was found that AM was associated with increased ossification. However, *in vivo* data of the present invention indicate the opposite. To understand this discrepancy we must find an indirect mechanism to explain what we observed. Our hypothesis is that AM reduces insulin levels, increasing the concentration of blood glucose. Glycemia regulates ghrelin levels and this, in turn, modulates bone homeostasis.

To measure the influence of the lack of AM in the regulation of glycemia, the animals were subjected to a glucose tolerance test: after 6 hours of fasting, basal glucose levels are established from a drop of blood taken from the tail vein and measured with a clinical monitor (Freestyle Optium, Abbott, Madrid). Next, glucose was injected intraperitoneally at a dose of 2 g/kg of weight. Glucose concentrations were measured at 15, 30, 45, 60 and 120 minutes. A commercial kit was used to measure the concentration of ghrelin by ELISA (Millipore).

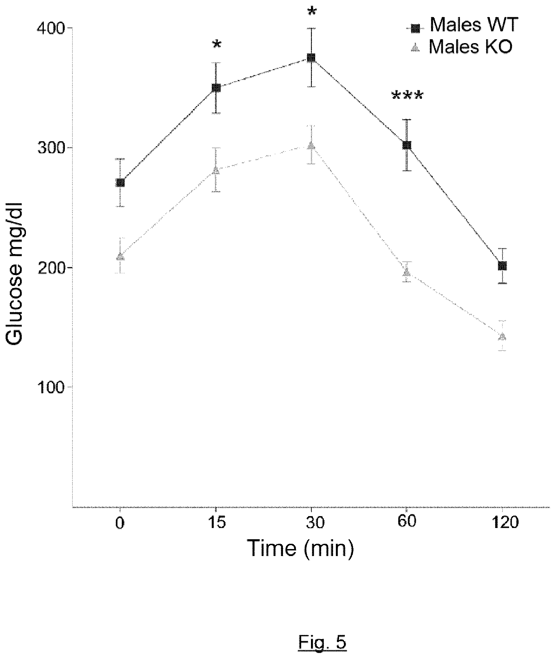

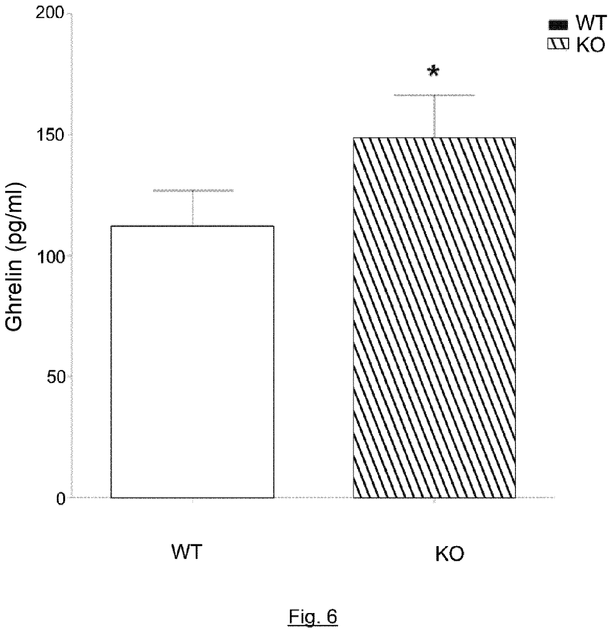

KO animals showed lower glucose levels than wild type animals (Fig. 5), a result that coincides with what it was expected, since AM was described as a tonic inhibitor of insulin secretion. When the levels of AM decrease in the KO, increased insulin secretion is expected and, therefore, lower glucose levels. On the other hand, when analyzing the blood levels of ghrelin, a higher concentration of this peptide is observed in the KO animals (Fig. 6). This result is also in line with what it was expected after studying the literature, since a reduction in glycemia results in increased secretion of ghrelin from the stomach into the blood.

### Example 3. Treatment of ovariectomized females with an inhibitor of the action of AM (molecule 16311).

An animal model of this disease was used. It is well known that, in female mice, the reduction of estrogen produced by ovariectomy results in symptoms of bone loss very similar to osteoporosis observed in postmenopausal women (Pang et al. 2014). For this, 40 wild genotype mice from the genetic pool C57BL/6 were used. Half of them (n = 20) were ovariectomized while the other half (n = 20) were subjected to the same surgery but the ovary (sham group) was not removed. Each of the groups was divided into two subgroups (10 mice each), one of which was injected with 100 µl of PBS 3 times a week for 4 weeks (control group) while the other received the same injections of molecule 16311 at a concentration of 3 ng/kg in 100 µl of PBS (treated group). At the end of the experiment blood samples were taken to study whether there had been any toxicity caused by the experimental molecule and the bone density of the femur was measured with microCT. Bone density parameters are expressed as the ratio between the ossified volume divided by the total volume of bone.

A clear decrease in bone density in the femur of ovariectomized animals (Fig. 7, group 3) when compared with sham group animals (group 1) was found. The most interesting is that the ovariectomized animals that received the treatment with the molecule 16311 (group 4) had significantly higher bone density (p<0.05) than the ovariectomized control group (group 3) and indistinguishable from that observed in the non-ovariectomized group (group 1), indicating that this molecule prevents the development of osteoporosis due to estrogen deprivation. Moreover there was no change in the sham mice that received the molecule (group 2).

On the other hand, it was studied whether chronic treatment with molecule 16311 caused any type of liver, kidney, or blood toxicity. As can be seen in Table 1, there is no significant difference among the parameters measured in female mice treated or untreated with said molecule, indicating a lack of toxicity for this compound, at least in mice.

**Table 1. Biochemical and blood count parameters in ovariectomized mice (OVX) receiving vehicle (PBS, first column) or the inhibitor (second column). None of the differences are statistically significant (p>0.05).**

| | | OVX + PBS | OVX + INHIBITOR |
|---|---|---|---|
| **BIOCHEMISTRY** | **UREA (mg/dl)** | 18.2 ± 3.33 | 18.5 ±3.78 |
| | **CREATININE (mg/dl)** | 0.132 ± 0.01 | 0.126 ± 0.02 |
| | **ALT (IU/l)** | 39.5 ± 2.33 | 42.8 ± 7.08 |
| | **AST (IU/l)** | 92 ± 5.62 | 82.2 ± 7.75 |
| | **ALKP (IU/l)** | 40 ± 34.27 | 43.6 ± 35.63 |
| | **GGT (IU/l)** | 1.14 ± 0.58 | 0.56 ± 0.36 |
| **BLOOD COUNT** | **VCA (%)** | 31.4 ± 0.33 | 30.58 ± 0.31 |
| | **GR (10⁶/µl)** | 7.67 ± 0.08 | 7.46 ± 0.09 |
| | **Hb (g/dl)** | 12.18 ± 0.15 | 11.78 ± 0.17 |
| | **GB (10³/µl)** | 6.10 ± 0.64 | 4.96 ± 0.68 |

## Claims

1. An inhibitor of adrenomedullin or an inhibitor of adrenomedullin receptors for use in the prevention and treatment of diseases that reduce bone density in a human or animal, **characterized in that** said inhibitor is selected from the group consisting of the compounds of formula: 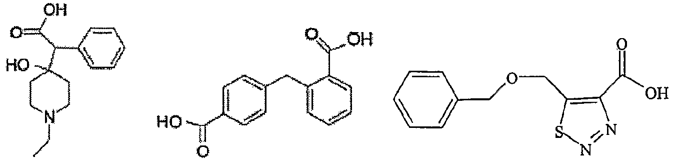 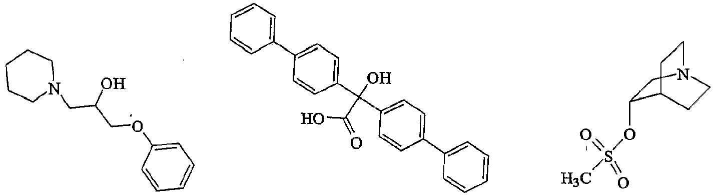 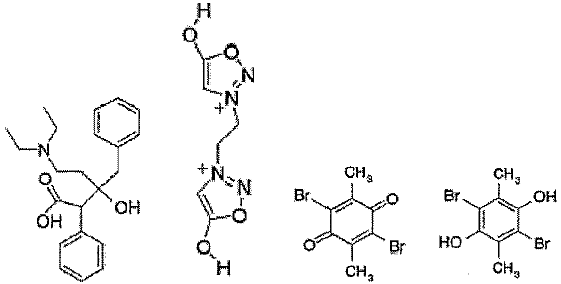 or is an interference RNA molecule, which comprises a sequence complementary to SEQ ID NO: 2 or SEQ ID NO: 3.

2. An inhibitor for use according to claim 1, **characterized in that** said disease is selected from the group consisting of osteoporosis, osteomalacia, rheumatoid arthritis, chronic kidney disease, hyperparathyroidism, Cushing's disease, cystic fibrosis, eating disorders, gastric bypass and prolonged immobility.

3. An inhibitor for use according to any of claims 1 to 2, **characterized in that** said inhibitor is used in combination with drugs selected from the group consisting of drugs that decrease bone mass or density, drugs that hormonally block prostate or breast cancer, drugs for treating seizures or epilepsy, heparin and glucocorticoids.

4. An inhibitor for use according to any of claims 1 to 3, **characterized in that** said animal is a farm animal or domestic animal.

5. An inhibitor for use according to any of claims 1 to 3, **characterized in that** the decrease in bone mass in a human is due to a state of weightlessness.

## Patentansprüche

1. Inhibitor von Adrenomedullin oder Inhibitor von Adrenomedullin-Rezeptoren zur Verwendung in der Prävention und Behandlung von knochendichtenverringernden Krankheiten bei Mensch oder Tier, **dadurch gekennzeichnet, dass** der genannte Inhibitor ausgewählt ist aus der Gruppe bestehend aus den Verbindungen der Formel: 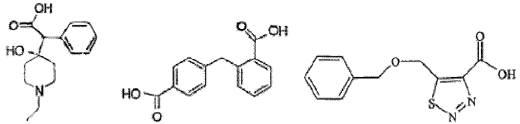 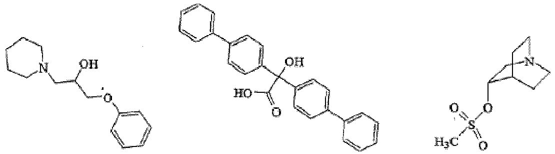 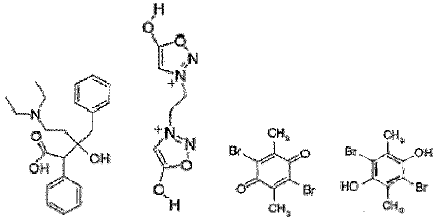 oder ein RNA-Interferenz-Molekül ist, das eine Sequenz umfasst, die zu Seq.-ID Nr. 2 oder Seq.-ID Nr. 3 komplementär ist.

2. Inhibitor zur Verwendung nach Anspruch 1, **dadurch gekennzeichnet, dass** die genannte Krankheit ausgewählt ist aus der Gruppe bestehend aus Osteoporose, Osteomalazie, rheumatoider Arthritis, chronischer Nierenerkrankung, Hyperparathyreoidismus, Cushing-Syndrom, Mukoviszidose, Essstörungen, Magenbypass und längere Bewegungslosigkeit.

3. Inhibitor zur Verwendung nach einem der Ansprüche 1 bis 2, **dadurch gekennzeichnet, dass** der genannte Inhibitor in Kombination mit Medikamenten verwendet wird, die ausgewählt sind aus der Gruppe bestehend aus Medikamenten, die Knochenmasse oder -dichte verringern, Medikamenten, die Prostata- oder Brustkrebs hormonell blockieren, Medikamenten zur Behandlung von Anfällen oder Epilepsie sowie Heparin und Glukokortikoiden.

4. Inhibitor zur Verwendung nach einem der Ansprüche 1 bis 3, **dadurch gekennzeichnet, dass** das genannte Tier ein Nutztier oder Haustier ist.

5. Inhibitor zur Verwendung nach einem der Ansprüche 1 bis 3, **dadurch gekennzeichnet, dass** der Abbau der Knochenmasse bei einem Menschen auf einen Zustand der Gewichtslosigkeit zurückzuführen ist.

## Revendications

1. Inhibiteur d'adrénomédulline ou inhibiteur des récepteurs d'adrénomédulline pour son utilisation dans la prévention et le traitement de maladies qui réduisent la densité osseuse chez l'humain ou l'animal, **caractérisé en ce que** ledit inhibiteur est choisi dans le groupe constitué par les composés de la formule: 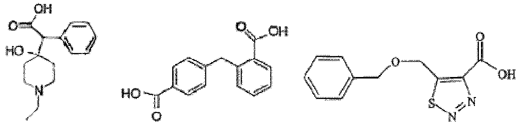 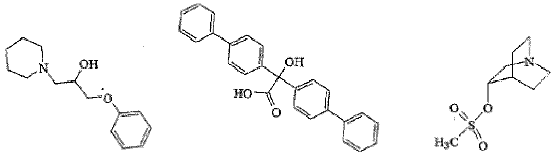 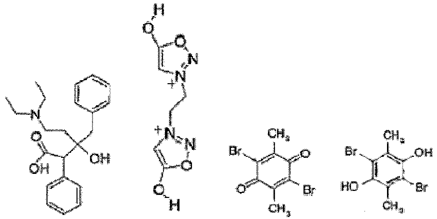 ou c'est une molécule d'ARN interférent, qui comprend une séquence complémentaire de SEQ ID NO: 2 or SEQ ID NO: 3.

2. Inhibiteur pour son utilisation selon la revendication 1, **caractérisé en ce que** ladite maladie est choisie dans le groupe constitué par l'ostéoporose, l'ostéomalacie, l'arthrite rhumatoïde, la maladie rénale chronique, l'hyperparathyroïdie, la maladie de Cushing, la fibrose kystique, les troubles alimentaires, le pontage gastrique et l'immobilité prolongée.

3. Inhibiteur pour son utilisation selon l'une quelconque des revendications 1 à 2, **caractérisé en ce que** ledit inhibiteur est utilisé en combinaison avec des médicaments choisis dans le groupe constitué par les médicaments qui diminuent la masse ou la densité osseuse, les médicaments qui bloquent hormonalement le cancer de la prostate ou du sein, les médicaments pour traiter les crises ou l'épilepsie, l'héparine et les glucocorticoïdes.

4. Inhibiteur pour son utilisation selon l'une quelconque des revendications 1 à 3, **caractérisé en ce que** ledit animal est un animal de ferme ou un animal domestique.

5. Inhibiteur pour son utilisation selon l'une quelconque des revendications 1 à 3, **caractérisé en ce que** la diminution de la masse osseuse chez l'humain est due à un état d'apesanteur.
